# EUROPEAN PATENT SPECIFICATION

(11) **EP 4 570 307 B1**
(45) Date of publication and mention of the grant of the patent: **04.11.2026**
(21) Application number: 25166390.2
(22) Date of filing: 09.03.2020
(51) Int. Cl.: A61N 1/05, A61N 1/36, A61N 1/372

(54) **IMPLANTABLE NEUROSTIMULATOR TO TREAT CHRONIC MIGRAINE HEADACHES**
IMPLANTIERBARER NEUROSTIMULATOR ZUR BEHANDLUNG VON CHRONISCHEM MIGRÄNEKOPFSCHMERZ
NEUROSTIMULATEUR IMPLANTABLE POUR TRAITER LES CÉPHALÉES MIGRAINEUSES CHRONIQUES

(30) Priority: 13.03.2019 US 201962817989 P
(43) Date of publication of application: 18.06.2025
(62) Document number(s) of the earlier application(s) in accordance with Art. 76 EPC: 20716316.3 / 3 938 026
(73) Proprietor: SHIRATRONICS, INC., Brooklyn Park MN 55455 (US)
(72) Inventor: JAIN, Mudit K., Santa Clara, CA 95054 (US)
(74) Representative: Murgitroyd & Company

(56) References cited:
- WO-A2-2017/049292
- US-A1- 2016 331 953

## Description

### TECHNICAL FIELD

This document relates generally to medical devices, and more particularly, but not by way of limitation, to systems, devices, and methods to devices, systems and associated methods for treating chronic headaches such as chronic migraine headaches.

### BACKGROUND

The treatment of chronic migraine has been limited to a number of procedures, pharmacological procedures or neuro-stimulation procedures. With respect to the neuro- stimulation procedures, devices have been developed for implanting in association with electrode leads, such that the electrodes are disposed proximate to some particular neural structure in an individual. These implanted devices can have batteries associated therewith for powering the electrodes or they can be externally activated.
WO2017049292 relates to devices and methods for peripheral nerve stimulation with an electrode assembly having a lead body with a tapered transition, at least one anchor, and at least one distal lead configured to connect to the lead body.

### SUMMARY

The invention is defined in the appended claims.

This document discusses, among other things, systems and methods to treat chronic headaches. The use of a single pulse generator to target more than one neural target around the head reduces surgical incisions and shortens the time to perform the implant procedure.

In one example, a neurostimulator system may include an implantable pulse generator with a processor, a driving system for driving electrodes, and a communication circuit,. At least one neurostimulation lead is provided having a plurality of electrodes disposed thereon and arranged in an array on the surface thereof, and one neurostimulator and lead having a plurality of branches, each branch radiating outward with each branch having at least one array of electrodes disposed on the external surface thereof.

This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates, by way of example, an implanted neurostimulation system.
FIG. 2 illustrates, by way of example, a lead array with multiple branching leads.
FIG. 3 illustrates, by way of example, an embodiment for subcutaneously locating the branching leads proximate to specific nerve bundles about the cranium of an individual.
FIG. 4 illustrates, by way of example, an alternate embodiment of FIG. 3 for subcutaneous placement of the IPG.
FIG. 5 illustrates, by way of example, an alternative implanted neurostimulation system.
FIG. 6 illustrates, by way of example, an embodiment for subcutaneously locating the neurostimulation system of FIG. 5 about the cranium of an individual.
FIG. 7 illustrates, by way of example, a side view of a stimulation lead.
FIG. 8 illustrates, by way of example, an embodiment depicting the subcutaneous placement of the lead of FIG. 7 proximate to nerve bundles about the cranium of an individual.
FIG. 9 illustrates, by way of example, an alternate embodiment of the subcutaneous placement of the leads of FIG. 8 with an alternate placement of the IPG.
FIG. 10 illustrates, by way of example, an alternate embodiment of the subcutaneous placement of the IPG and the associated leads about the cranium of an individual.

### DETAILED DESCRIPTION

The following detailed description should be read with reference to the drawings. These drawings, which are not necessarily to scale, depict illustrative embodiments and are not intended to limit the scope of the invention.

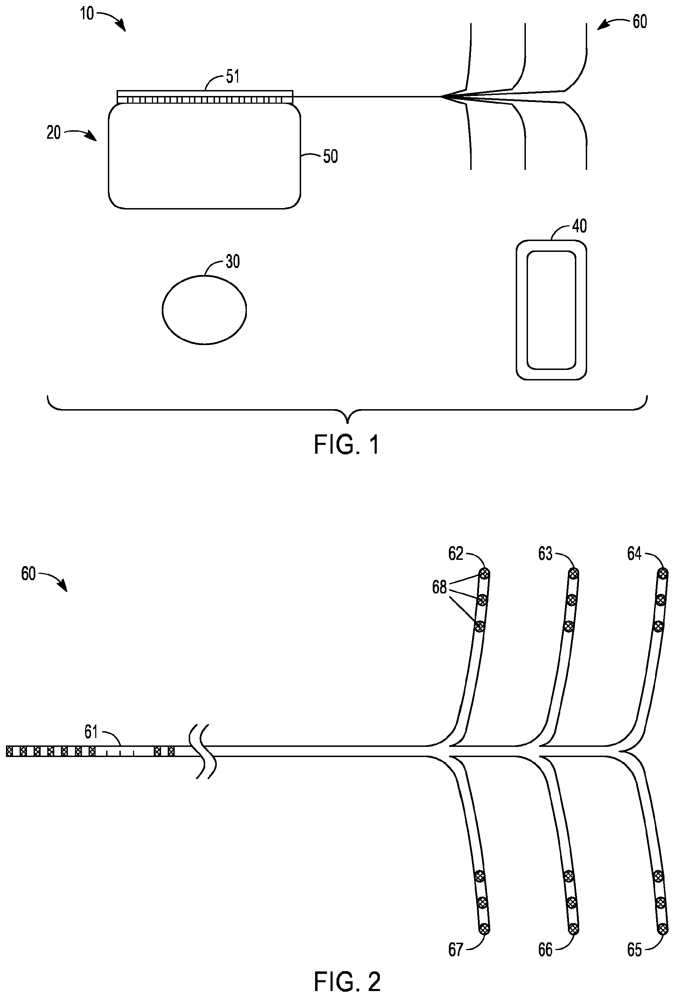

FIG. 1 illustrates an implanted neurostimulation system 10 including an implanted system 20, an external telemetry system and charging system 30, and a remote controller 40. The implanted system 20 may generally include an implanted pulse generator (IPG) 50, and implanted stimulation lead system 60. The IPG 50 may include a header 51 for connection with the stimulation leads 60, and a hermetically insulated housing for electronics and non-chargeable or rechargeable battery (not shown), which electronics may include a processor, a driving circuit for driving stimulation leaves and a communication circuit for communicating external to the IPG 50, a communication circuit comprising, without limitation, a near field conductive communication system. The stimulation lead(s) 60 may include a lead body with proximal connector and distal branches with nerve stimulation electrodes.

FIG. 2 illustrates a stimulation lead system 60 configured to be subcutaneously implanted. By way of example and not limitation, the stimulation lead system may include six distal lead branches 62 - 67, which are shown schematically and not to scale. The proximal connectors 61 may include individual connectors that provide electrical connection between stimulation output channels of the IPG 50 embedded in the header 51 to the stimulation electrodes 68 on lead branch 62. Each distal lead branch may, for example, include two to three stimulation electrodes. The system may include, by way example and not limitation, 6 to18 individual proximal connectors to provide electrical connection to the electrodes.

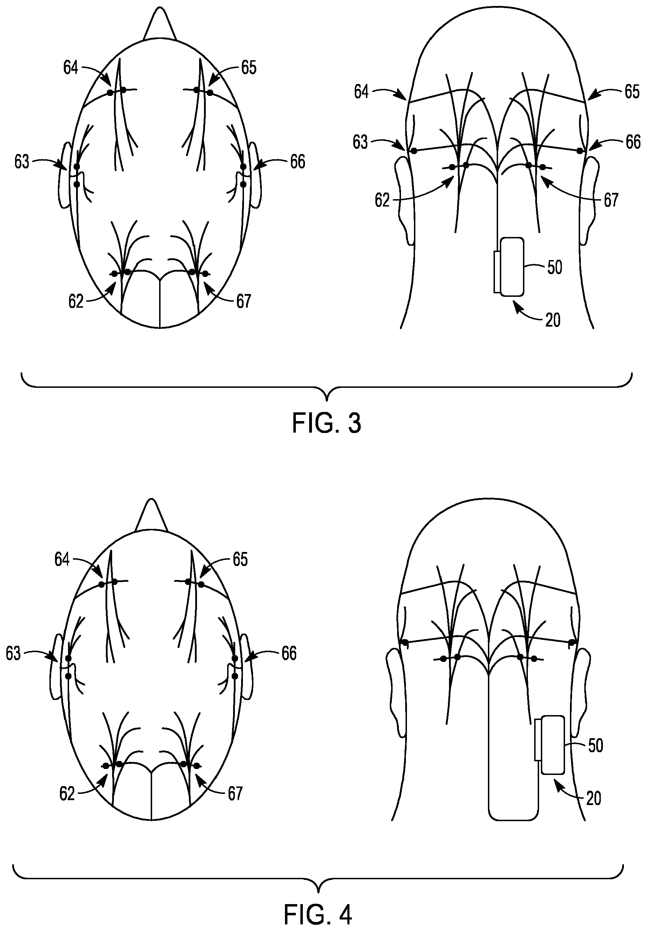

FIG. 3 illustrates, by way of example and not limitation, the implanted system 20 of the IPG 50 and implanted stimulation lead system 60 subcutaneously implanted in a patient. In the illustrated embodiment, the stimulation lead system 60 with its distal lead branches 62 - 67 and their stimulation electrodes are subcutaneously disposed on six groups of pre-determined location of nerve branches (left and right occipital nerves, left and right temporal nerves, and left and right supraorbital nerves). The IPG 50 may be implanted in a subcutaneous pocket generally in the area superficial to the splenius capitis muscle or semispinalis capitis muscle near the upper cervical region.

An alternative location for the IPG 50 with lead system 60, as shown in FIG. 4, may be a subcutaneous pocket superficial to the sternocleidomastoid muscle inferior to the mastoid process. In the illustrated embodiments, the lead generally ascends along a midline of the head, providing symmetrical distribution to neural targets on the left and right side of the head.

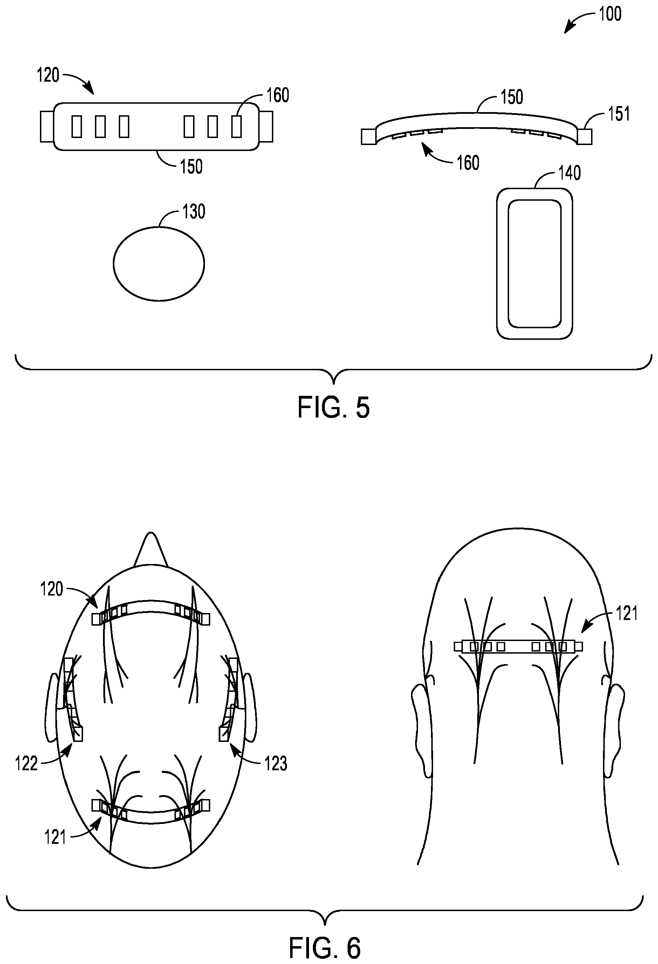

FIG. 5 illustrates an alternative subcutaneously implanted neurostimulation system 100 including an implanted system 120, an external telemetry system 130 and charging system 131, and remote controller 140 is shown schematically. The implanted system 120 may generally include an implanted pulse generator (IPG) 150, and stimulation electrodes 160 embedded in the housing of IPG 150. The IPG 150, insulated hermetically, contains electronics and non-chargeable or rechargeable battery and four to six stimulation electrodes 160 embedded in the housing.

FIG. 6 illustrates, by way of example and not limitation, four subcutaneously implanted neurostimulation systems 100 implanted in a patient. One implanted system 120 may be placed with its stimulation electrodes disposed superficial to the left and right supraorbital nerves. One implanted system 121 may be placed with its stimulation electrode disposed superficial to the left and right occipital nerves. One implanted system 122 may be placed with its stimulation electrode disposed superficial to the left temporal nerves. One implanted system 123 may be placed with its stimulation electrode disposed superficial to the right temporal nerves.

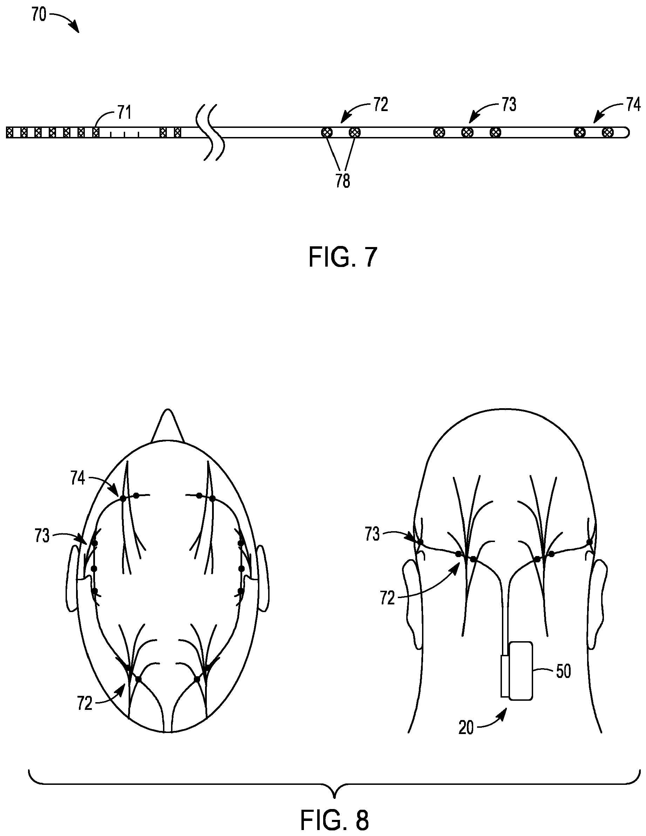

FIG. 7 illustrates an implanted stimulation lead system 70 including one lead body with three electrode sets or groups 72 - 74, which is shown schematically and not to scale. The proximal connectors 71 may include of 3 to 9 individual connectors that provide electrical connection between stimulation output channels of the IPG 50 embedded in the header 51 to the stimulation electrodes 78 as a part of electrode group 72, as illustrated at one of the electrode group and represent similar configuration for other electrode groups. Generally, each distal lead branch may include an electrode set, where the electrode set may include two to three stimulation electrodes. Each of the stimulation electrodes can function as either a cathode or anode and the IPG and be programmed to configure the electrodes as anodes, cathodes or nonfunctional electrodes, depending upon the application.

FIG. 8 illustrates, by way of example and not limitation, the implanted system 20 of the IPG 50 and implanted stimulation lead system 70 subcutaneously implanted in a patient. The stimulation lead system 70 may have two leads, each with at least one electrode set or group and illustrated with three electrode sets or groups 72 - 74. The stimulation electrodes of each of the six electrode sets are disposed on six neural targets (e.g. nerve branches such as left and right occipital nerves, left and right temporal nerves, and left and right supraorbital nerves). The IPG 50 may be implanted in a subcutaneous pocket generally in the area superficial to the splenius capitis muscle or semispinalis capitis muscle near the upper cervical region.

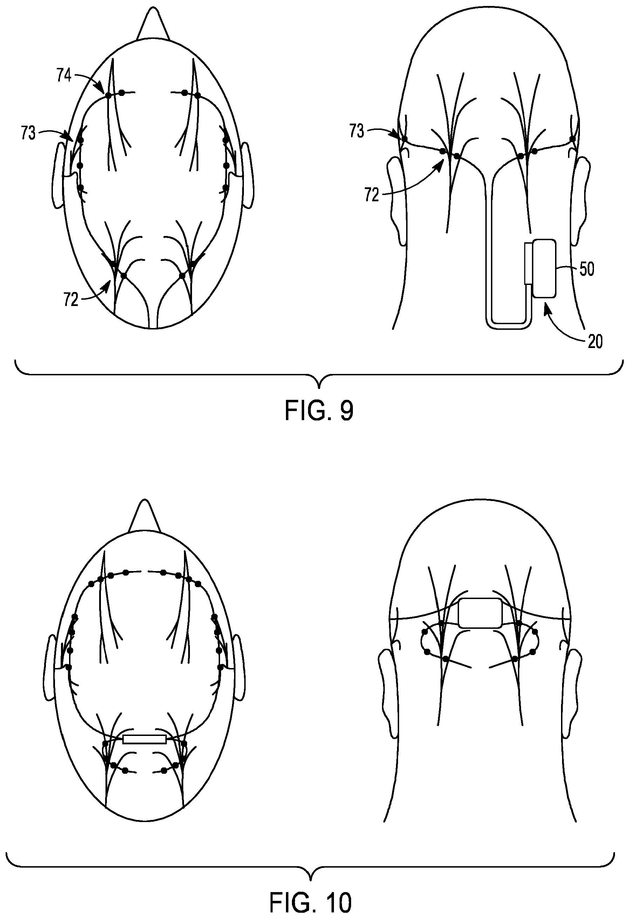

An alternative location for the IPG 50 with lead system 70, as shown in FIG. 9, may be a subcutaneous pocket superficial to the sternocleidomastoid muscle inferior to the mastoid process.

With reference to the systems and devices depicted in FIGS. 1 - 4, an implanting surgeon may place the electrode sets (e.g. the distal leads 62 - 67 of the stimulation leads system 60) near pre-determined nerve targets (e.g., left and right supraorbital, left and right temporal, and left and right occipital nerves) through subcutaneous tunneling after making one or multiple incisions. The implanting surgeon may then make a subcutaneous pocket either superficial to the sternocleidomastoid muscle or in a more medial location that is superficial to the semispinalis capitis muscle. The surgeon may then connect the stimulation lead 60 to the IPG 50 and place them in the subcutaneous pocket.

As indicated above, left-side nerve targets may include one or more of the following nerve targets: left supraorbital nerve, left temporal nerve, and left occipital nerve. Similarly, right-side nerve targets may include one or more of the following nerve targets: right supraorbital nerve, right temporal nerve, and right occipital nerve. One or more of these nerves may be targeted to provide a therapy to treat a headache such as a migraine headache. Other therapies may be delivered, and neuromodulation may be delivered to these or other neural target(s) as may be indicated to treat various conditions. A non-exclusive example of other neural target(s) includes combinations of one or more of a greater occipital nerve (GON), a supraorbital nerve (SON), and a sphenopalatine ganglion (SPG). Modulation of one or more of these neural targets may be useful to treat cluster headaches. Another non-exclusive example includes combinations of one or more of a supraorbital nerve (SON) and a facial nerve. Modulation of one or more of these neural targets may be useful to treat facial pain. The lead(s) are designed to extend from the implantable pulse generator to enable electrode sets to be positioned at these neural target(s).

With reference to an alternative system and devices depicted in FIGS. 7 - 9, the implanting surgeon may place the electrode sets (e.g. groups 72 - 74 of the stimulation leads system 70) near pre-determined nerve targets (e.g., supraorbital, temporal, and occipital nerves) with two lead systems to cover both the left and right side of these nerves.

The patient with the implanted system 20 or a physician can adjust stimulation output parameters using the remote controller 40 through an external telemetry system 30. The patient can recharge the implanted system with a rechargeable battery using the external charging system 31.

The IPG 50 may contain hardware and firmware that deliver independent output to each stimulation electrode. The stimulation output parameters consist of amplitude, polarity, frequency, pulse width and duty cycle.

The remote controller 40 may be configured (e.g. via software, to provide a graphic user interface used to make adjustments of stimulation output parameters. The remote controller 40 also contains a stimulation algorithm from user input or through machine learning. The stimulation algorithm may include time dependent of individual setting (amplitude, polarity, frequency, pulse width and duty cycle) for each stimulation electrode.

With reference to the systems and devices depicted in FIGS. 5-6, the implanting surgeon may place implanted system 120 through an incision directly over pre-determined nerve targets (e.g., left and right supraorbital, left and right temporal, and left and right occipital nerves). The surgeon may fix the implanted system 120 on nearby fascia with the suture rings on the two ends of the IPG 150.

The patient with the implanted system 120 or a physician can adjust stimulation output parameters using the remote controller 140 through an external telemetry system, and the patient may recharge the implanted system with a rechargeable battery using the external charging system. Both the eternal telemetry system and external charging system are illustrated by an external device identified using reference number 130.

The IPG 150 may contain hardware and firmware that deliver independent output to each stimulation electrode. The stimulation output parameters may include amplitude, clarity, frequency, pulse width and duty cycle.

The remote controller 140 may contain software that provides a graphic user interface for making adjustments of stimulation output parameters. The remote controller 140 may contain a stimulation algorithm from user input or through machine learning. The stimulation algorithm may include time dependent of individual setting (amplitude, frequency, clarity, pulse width and duty cycle) for each stimulation electrode.

FIG. 10 depicts embodiments that incorporate the above concepts into a single body device with no header. The lead(s) may be permanently attached to the pulse generator, and the pulse generator and lead(s) together may form a unibody construction where lead body (bodies) is (are) integrally formed with the pulse generator. For example, a lead body may be integrally formed with a housing of the pulse generator.

Although the preferred embodiment has been described in detail, it should be understood that various changes, substitutions and alterations can be made therein without departing from scope of the invention as defined by the appended claims.

A circuit or circuitry may be implemented as part of a microprocessor circuit, which may be a dedicated processor such as a digital signal processor, application specific integrated circuit (ASIC), microprocessor, or other type of processor for processing information including physical activity information. The microprocessor circuit may be a general purpose processor that may receive and execute a set of instructions of performing the functions, methods, or techniques described herein. The circuit or circuitry may be implemented as one or more other circuits or sub-circuits that may, alone or in combination, perform the functions, methods or techniques described herein. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified to encode instructions of the specific operation. Instructions may enable embedded hardware (e.g., the execution units or a loading mechanism) to create members of the circuit set in hardware via variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

The terms "tangible" and "non-transitory," as used herein, are intended to describe a machine-readable storage medium such as a computer-readable storage medium (or "memory") excluding propagating electromagnetic signals, but are not intended to otherwise limit the type of physical computer-readable storage device that is encompassed by the phrase computer-readable medium or memory. By way of example and not limitation, a machine may include a modulation device or a programming device such as a remote control or clinician programmer. For instance, the terms "non-transitory computer readable medium" or "tangible memory" are intended to encompass types of storage devices that do not necessarily store information permanently, including for example, random access memory (RAM).

The term "machine readable medium" may include a single medium or multiple media (e.g., a centralized or distributed database, and/or associated caches and servers) configured to store instructions, and includes any medium that is capable of storing, encoding, or carrying instructions for execution by the machine and that cause the machine to perform any one or more of the techniques of the present disclosure, or that is capable of storing, encoding or carrying data structures used by or associated with such instructions. Nonlimiting machine readable medium examples may include solid-state memories, and optical and magnetic media. In an example, a machine readable medium include: nonvolatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks. Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

Method examples described herein can be machine or computerimplemented at least in part. Some examples may include a computer-readable medium or machine-readable medium encoded with instructions operable to configure an electronic device or system to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times.

Various embodiments are illustrated in the figures above. One or more features from one or more of these embodiments may be combined to form other embodiments.

The above detailed description is intended to be illustrative, and not restrictive. The scope of the disclosure should, therefore, be determined with references to the appended claims.

## Claims

1. A neurostimulator system (10), comprising:
one implantable pulse generator (50) configured to be subcutaneously implanted and at least one implantable lead (60) configured to extend from the subcutaneously-implanted implantable pulse generator (50) to at least four neural targets including at least two neural targets on a left side of a head and at least two neural targets on a right side of the head, the at least four neural targets including left supraorbital nerves, left occipital nerves, right supraorbital nerves, and right occipital nerves, the at least one implantable lead (60) including:
a lead body with proximal connectors (61) and distal branches (62 - 67) including at least a first pair of right-side and left-side branches (62, 67) and a second pair of right-side and left-side branches (63, 66), each including nerve stimulation electrodes (68);
the proximal connectors (61) being configured to provide an electrical connection between the implantable pulse generator (50) and the stimulation electrodes (68);
the at least first and second pairs of right-side and left-side branches (62, 63, 66, 67) radiating outward from the lead body wherein:
the right-side and left-side branches (62, 63, 66, 67) are configured, in use, to stimulate right and left neural targets respectively.

2. The neurostimulator system according to claim 1, wherein each of the first pair of branches (62, 67) have a first length, and each of the second pair of branches (63, 66) have a second length different from the first length.

3. The neurostimulator system according to claim 2, wherein the implantable lead further includes a third pair of right-side and left-side branches (64, 65), and each of the third pair of right-side and left-side branches (64, 65) have a third length different from the first length and the second length.

4. The neurostimulator system according to any of claims 1-2, wherein each branch of the first pair of right-side and left-side branches (62, 67) and each branch of the second pair of right-side and left-side branches (63, 66) include at least two electrodes (68).

5. The neurostimulator system according to claim 4, wherein the at least two electrodes (68) include three electrodes (68).

6. The neurostimulator system according to any of claims 1-5, wherein a total number of nerve stimulation electrodes (68) correspond to a number of proximal connectors (61), and the implantable pulse generator (50) is configured to independently control stimulation from each of the total number of electrodes (68).

7. The neurostimulator system according to any of claims 1-6, wherein the implantable pulse generator (50) is configured for implantation in a subcutaneous pocket superficial to a splenius capitis muscle or a semispinalis capitis muscle or in a subcutaneous pocket superficial to a sternocleidomastoid muscle inferior to a mastoid process.

8. The neurostimulator system according to any of claims 1-7, wherein the at least four neural targets include left supraorbital nerves, left temporal nerves, left occipital nerves, right supraorbital nerves, right temporal nerves, and right occipital nerves.

9. The neurostimulator system according to any of claims 1-7, wherein the at least four neural targets include a greater occipital nerve (GON), a supraorbital nerve (SON), and a sphenopalatine ganglion (SPG).

10. The neurostimulator system according to any of claims 1-7, wherein the at least four neural targets include a facial nerve and a supraorbital nerve (SON).

## Patentansprüche

1. Ein Neurostimulatorsystem (10), das Folgendes beinhaltet:
einen implantierbaren Impulsgenerator (50), der konfiguriert ist, um subkutan implantiert zu werden, und mindestens eine implantierbare Leitung (60), die konfiguriert ist, um sich von dem subkutan implantierten implantierbaren Impulsgenerator (50) zu mindestens vier neuralen Zielen zu erstrecken, die mindestens zwei neurale Ziele auf einer linken Seite eines Kopfes und mindestens zwei neurale Ziele auf einer rechten Seite des Kopfes umfassen, wobei die mindestens vier neuralen Ziele linke supraorbitale Nerven, linke Occipitalnerven, rechte supraorbitale Nerven und rechte Occipitalnerven umfassen, wobei die mindestens eine implantierbare Leitung (60) Folgendes umfasst:
einen Leitungskörper mit proximalen Verbindern (61) und distalen Zweigen (62 - 67), die mindestens ein erstes Paar von rechtsseitigen und linksseitigen Zweigen (62, 67) und ein zweites Paar von rechtsseitigen und linksseitigen Zweigen (63, 66) umfassen, die jeweils Nervenstimulationselektroden (68) umfassen;
wobei die proximalen Verbinder (61) konfiguriert sind, um eine elektrische Verbindung zwischen dem implantierbaren Impulsgenerator (50) und den Stimulationselektroden (68) bereitzustellen;
wobei das mindestens erste und zweite Paar von rechtsseitigen und linksseitigen Zweigen (62, 63, 66, 67) von dem Leitungskörper nach außen strahlen, wobei:
die rechtsseitigen und linksseitigen Zweige (62, 63, 66, 67) im Gebrauch konfiguriert sind, um rechte bzw. linke neurale Ziele zu stimulieren.

2. Neurostimulatorsystem gemäß Anspruch 1, wobei jeder des ersten Paars von Zweigen (62, 67) eine erste Länge aufweist und jeder des zweiten Paars von Zweigen (63, 66) eine zweite Länge aufweist, die sich von der ersten Länge unterscheidet.

3. Neurostimulatorsystem gemäß Anspruch 2, wobei die implantierbare Leitung ferner ein drittes Paar von rechtsseitigen und linksseitigen Zweigen (64, 65) umfasst und jeder des dritten Paars von rechtsseitigen und linksseitigen Zweigen (64, 65) eine dritte Länge aufweist, die sich von der ersten Länge und der zweiten Länge unterscheidet.

4. Neurostimulatorsystem gemäß einem der Ansprüche 1-2, wobei jeder Zweig des ersten Paars von rechtsseitigen und linksseitigen Zweigen (62, 67) und jeder Zweig des zweiten Paars von rechtsseitigen und linksseitigen Zweigen (63, 66) mindestens zwei Elektroden (68) umfasst.

5. Neurostimulatorsystem gemäß Anspruch 4, wobei die mindestens zwei Elektroden (68) drei Elektroden (68) umfassen.

6. Neurostimulatorsystem gemäß einem der Ansprüche 1-5, wobei eine Gesamtanzahl von Nervenstimulationselektroden (68) einer Anzahl von proximalen Verbindern (61) entspricht und der implantierbare Impulsgenerator (50) konfiguriert ist, um die Stimulation von jeder der Gesamtanzahl von Elektroden (68) unabhängig zu steuern.

7. Neurostimulatorsystem gemäß einem der Ansprüche 1-6, wobei der implantierbare Impulsgenerator (50) zur Implantation in eine subkutane Tasche, die oberflächlich zu einem Splenius-capitis-Muskel oder einem Semispinalis capitis-Muskel ist, oder in eine subkutane Tasche, die oberflächlich zu einem Sternocleidomastoideus-Muskel ist, der einem Mastoideusprozess nachgeordnet ist, konfiguriert ist.

8. Neurostimulatorsystem gemäß einem der Ansprüche 1-7, wobei die mindestens vier neuralen Ziele linke supraorbitale Nerven, linke Temporalnerven, linke Occipitalnerven, rechte supraorbitale Nerven, rechte Temporalnerven und rechte Occipitalnerven umfassen.

9. Neurostimulatorsystem gemäß einem der Ansprüche 1-7, wobei die mindestens vier neuralen Ziele einen Nervus occipitalis major (GON), einen supraorbitalen Nerv (SON) und ein Ganglion pterygopalatinum (SPG) umfassen.

10. Neurostimulatorsystem gemäß einem der Ansprüche 1-7, wobei die mindestens vier neuralen Ziele einen Gesichtsnerv und einen supraorbitalen Nerv (SON) umfassen.

## Revendications

1. Un système de neurostimulateur (10), comprenant :
un générateur d'impulsions implantable (50) configuré pour être implanté de façon sous-cutanée et au moins une sonde implantable (60) configurée pour s'étendre depuis le générateur d'impulsions implantable (50) implanté de façon sous-cutanée jusqu'à au moins quatre cibles neurales incluant au moins deux cibles neurales sur un côté gauche de la tête et au moins deux cibles neurales sur un côté droit de la tête, les au moins quatre cibles neurales incluant des nerfs supraorbitaires gauches, des nerfs occipitaux gauches, des nerfs supraorbitaires droits et des nerfs occipitaux droits, l'au moins une sonde implantable (60) incluant :
un corps de sonde présentant des connecteurs proximaux (61) et des branches distales (62 - 67) incluant au moins une première paire de branches pour côté droit et côté gauche (62, 67) et une deuxième paire de branches pour côté droit et côté gauche (63, 66), chacune incluant des électrodes de stimulation nerveuse (68) ;
les connecteurs proximaux (61) étant configurés pour établir une connexion électrique entre le générateur d'impulsions implantable (50) et les électrodes de stimulation (68) ;
les première et deuxième paires, au moins, de branches pour côté droit et côté gauche (62, 63, 66, 67) rayonnant vers l'extérieur à partir du corps de sonde, dans lequel :
les branches pour côté droit et côté gauche (62, 63, 66, 67) sont configurées, lors de l'utilisation, pour stimuler des cibles neurales droites et gauches respectivement.

2. Le système de neurostimulateur selon la revendication 1, dans lequel chaque branche de la première paire de branches (62, 67) présente une première longueur, et chaque branche de la deuxième paire de branches (63, 66) présente une deuxième longueur différente de la première longueur.

3. Le système de neurostimulateur selon la revendication 2, dans lequel la sonde implantable inclut en outre une troisième paire de branches pour côté droit et côté gauche (64, 65), et chaque branche de la troisième paire de branches pour côté droit et côté gauche (64, 65) présente une troisième longueur différente de la première longueur et de la deuxième longueur.

4. Le système de neurostimulateur selon n'importe lesquelles des revendications 1 à 2, dans lequel chaque branche de la première paire de branches pour côté droit et côté gauche (62, 67) et chaque branche de la deuxième paire de branches pour côté droit et côté gauche (63, 66) incluent au moins deux électrodes (68).

5. Le système de neurostimulateur selon la revendication 4, dans lequel les au moins deux électrodes (68) incluent trois électrodes (68).

6. Le système de neurostimulateur selon n'importe lesquelles des revendications 1 à 5, dans lequel un nombre total d'électrodes de stimulation nerveuse (68) correspond à un nombre de connecteurs proximaux (61), et le générateur d'impulsions implantable (50) est configuré pour commander indépendamment la stimulation issue de chaque électrode parmi le nombre total d'électrodes (68).

7. Le système de neurostimulateur selon n'importe lesquelles des revendications 1 à 6, dans lequel le générateur d'impulsions implantable (50) est configuré pour une implantation dans une poche sous-cutanée superficielle à un muscle splénius de la tête ou à un muscle semi-épineux de la tête ou dans une poche sous-cutanée superficielle à un muscle sterno-cléido-mastoïdien inférieur à un processus mastoïde.

8. Le système de neurostimulateur selon n'importe lesquelles des revendications 1 à 7, dans lequel les au moins quatre cibles neurales incluent des nerfs supraorbitaires gauches, des nerfs temporaux gauches, des nerfs occipitaux gauches, des nerfs supraorbitaires droits, des nerfs temporaux droits, et des nerfs occipitaux droits.

9. Le système de neurostimulateur selon n'importe lesquelles des revendications 1 à 7, dans lequel les au moins quatre cibles neurales incluent un nerf grand occipital (NGO), un nerf supraorbitaire (NSO), et un ganglion sphénopalatin (GSP).

10. Le système de neurostimulateur selon n'importe lesquelles des revendications 1 à 7, dans lequel les au moins quatre cibles neurales incluent un nerf facial et un nerf supraorbitaire (NSO).
